(19) Europäisches Patentamt
European Patent Office
Office européen des brevets

(11) Veröffentlichungsnummer: **0 108 322**
**B1**

(12) # EUROPÄISCHE PATENTSCHRIFT

(45) Veröffentlichungstag der Patentschrift: **29.01.86**

(51) Int. Cl.⁴: **C 07 C 43/313**, C 07 C 41/48, C 07 D 249/08

(21) Anmeldenummer: **83110596.0**

(22) Anmeldetag: **24.10.83**

(54) **Keten-0,0-acetale und Verfahren zu ihrer Herstellung.**

(30) Priorität: **30.10.82 DE 3240287**

(43) Veröffentlichungstag der Anmeldung:
**16.05.84 Patentblatt 84/20**

(45) Bekanntmachung des Hinweises auf die Patenterteilung:
**29.01.86 Patentblatt 86/5**

(84) Benannte Vertragsstaaten:
**AT BE CH DE FR GB IT LI NL SE**

(56) Entgegenhaltungen:
**EP-A-0 110 116**

**THE JOURNAL OF ORGANIC CHEMISTRY, Band 44, Nr. 3, Seiten 356-359, 2. February 1979 W. ADAM et al.: "Synthesis of Ketene Diphenyl Acetals via Decarboxylation of beta-Lactones Derived from the Lithium alpha, alpha-Dipehnoxy-alpha-lithioacetate Synthon"**

(73) Patentinhaber: **BASF Aktiengesellschaft, Carl-Bosch-Strasse 38, D-6700 Ludwigshafen (DE)**

(72) Erfinder: **Ruland, Alfred, Dr., Schriesheimer Strasse 11, D-6945 Hirschberg (DE)**
Erfinder: **Reuther, Wolfgang, Dr., Am Pferchelhang 16, D-6900 Heidelberg (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Keten-O,O-acetale und Verfahren zu ihrer Herstellung durch Abspaltung von Wasser aus α-Hydroxyacetalen.

Zur Herstellung von Keten-O,O-acetale sind mehrere Verfahren bekannt (D. Borrmann in Houben-Weyl-Müller, Methoden der organischen Chemie, Bd. 7/4, S. 340 ff., Thieme Verlag, Stuttgart 1968):

1. Abspaltung von Halogenwasserstoff aus α-Halogenacetalen mit Alkalialkoholen insbesondere Kalium-tert.-butanolat. Dieses Verfahren hat jedoch einige Nachteile. Zum einen sind damit in mässigen bis mittleren Ausbeuten nur unsubstituierte oder einfach mit Chlor, Brom oder Phenyl substituierte Keten-O,O-acetale zugänglich, während z. B. die Ausbeute beim Isopropyl-ketendiethylacetal bereits auf 22% absinkt. Zum anderen handelt es sich bei den Alkoholkomponenten praktisch ausschliesslich um einfache aliphatische Alkohole wie Methanol und Ethanol. Weiterhin sind die zur Synthese höher substituierter Keten-O,O-acetale mit Phenolen als OR-Komponente benötigten α-Halogenacetale bisher nicht bekannt und aufgrund verschiedener Nebenreaktionen nur schwer zugänglich.

2. Ein weiteres bekanntes Verfahren besteht in der Abspaltung von Alkoholen aus Orthocarbonsäuretriestern. Auch dieses Verfahren ist im Hinblick auf die Darstellung der benötigten Orthocarbonsäureester mit Phenolen als Alkoholkomponente praktisch nicht einsetzbar. Gemischte Keten-O,O-acetale sind auf diesem Wege ebenfalls nicht kontrolliert zugänglich. Die gleichen Einschränkungen gelten auch für die Synthese von Keten-O,O-acetalen durch Abspaltung von Alkylhypobromiden aus α-Bromcarbonsäuretriestern.

3. Umsetzungen von 1,1-Dihaloethylenen zu Keten-O,O-acetalen sind ebenfalls bekannt. Sie gelingen aber nur bei β-aktivierten Ethylenen oder im speziellen Fall des 1,1-Dichlorethylens durch Umsetzung mit β- oder γ-Alkoxy- bzw. Dialkylaminoalkoholaten.

Aus J. Org. Chem. *44*/3, 356-359 (1979) ist es bekannt, Keten-Diphenyl-Acetale herzustellen. Es findet sich jedoch kein Hinweis auf die neuen Verbindungen gemäss der Erfindung oder auf das Verfahren zu ihrer Herstellung.

Es wurde nun gefunden, dass Keten-O,O-acetale der Formel I:

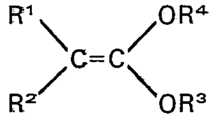 (I)

in der

$R^1$ einen tertiären Alkylrest mit 4 bis 6-Atomen oder einen durch Halogen substituierten Phenylrest,

$R^2$ Wasserstoff,

$R^3$ und $R^4$ unabhängig voneinander einen gegebenenfalls durch Halogen, Alkoxy, Alkyl, Phenyl, Phenoxy, Cyano, Nitro, Trifluormethyl substituierten Phenylrest bedeuten, wertvolle Verbindungen sind, die sich hervorragend eignen zur Herstellung der bekannten (DE-OS Nr. 3100261) Keten-O,N-acetale der Formel IV:

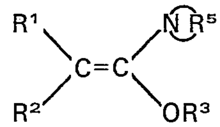 (IV)

in der

$R^1$ bis $R^3$ die im Anspruch 1 genannten Bedeutungen haben und $-N\ R^5$ einen Triazolyl-, Imidazolyl-, Benzimidazolyl- oder Pyrazolylrest bedeutet;

$R^1$ bedeutet beispielsweise einen tertiär-Butylrest;

$R^3$ bedeutet beispielsweise einen durch Halogen (F, Cl, Br), Alkoxy mit 1 bis 2 C-Atomen (Methoxy), Alkyl mit 1 bis 4 C-Atomen (Methyl, Ethyl, Propyl, i-Propyl, Butyl, sek.-Butyl, tert.-Butyl, i-Butyl) substituierten Phenylrest, wobei einzelne Reste mehrfach (2-3fach) oder verschiedene Reste gleichzeitig als Substituenten auftreten, z. B. 4-Chlorphenyl, 3,4-Dichlorphenyl, 4-Chlor-3-bromphenyl, 4-Methyl-3-chlorphenyl,

$R^4$ hat beispielsweise unabhängig von $R^3$ die gleichen Bedeutungen wie $R^3$ $-N\ R^5$ bedeutet beispielsweise 1-(1,2,4)-Triazolyl, 1-(1,2,3-Triazolyl).

Die neuen Keten-O,O-acetale der Formel I werden beispielsweise hergestellt, indem man:

a) ein α-Hydroxyacetal der Formel II:

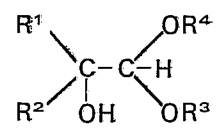 (II)

in der

$R^1$ bis $R^4$ die im Anspruch 1 genannten Bedeutungen haben mit der Verbindung:

$$Cl-Z$$

zu der Verbindung der Formel III:

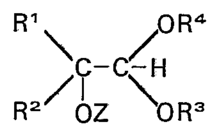 (III)

umsetzt, in der Z den Rest $-SO_2-R$ bedeutet, wobei R Alkyl ($C_1$-$C_4$), Haloalkyl ($C_1$-$C_4$-) oder gegebenenfalls einfach oder mehrfach (z. B. durch Alkyl ($C_1$-$C_4$)) substituiertes Phenyl bedeutet,

b) und die Verbindung der Formel III mit einer basischen Verbindung zu dem Keten-O,O-acetale der Formel I gemäss Anspruch 1 umsetzt.

Durch Reduktion der Ketone der Formel V:

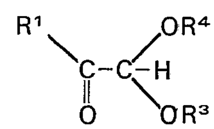 (V)

erhält man beispielsweise die α-Hydroxyacetale der Formel II.

Die Ketone der Formel V sind bekannt oder sie können nach bekannten Verfahren hergestellt werden (vgl. z. B. J. Chem. Soc., 1970, 462-464, Liebigs Ann. Chem. *735*, 145 (1970).

Die Umsetzung der Verbindung der Formel III mit einer basischen Verbindung erfolgt beisipelsweise in aprotisch dipolaren Lösungsmitteln, wie Dimethylsulfoxid, Dimethylformamid oder Hexamethylphosphorsäuretriamid mit basischen Verbindungen, wie Alkali- und Erdalkalihydroxiden. Alkali- und Erdalkalisulfiden, Alkali-, Erdalkali- und Aluminiumalkoholaten bei Temperaturen zwischen 20 und 150°C. Das Verfahren hat den grossen Vorteil, dass die Ketone der Formel V leicht aus α-Halogen- bzw. α,α-Dihalogenketonen zugänglich sind, wobei der Variation der OR-Reste praktisch keine Grenzen gesetzt sind. Durch sukzessive Einführung der Reste $OR^3$ und $OR^4$ lassen sich beliebige Kombinationen herstellen. Die Reduktion der Ketone der Formel V ist bekannt. Bei den Verbindungen der Formel III handelt es sich um Verbindungen, die nach den üblichen Methoden, z. B. aus Alkalialkoholaten und Sulfonsäurehalogeniden in guten bis sehr guten Ausbeuten gewonnen werden können.

Überraschend ist ferner die glatte Abspaltung der Gruppe –OZ (Z = z. B. $SO_2$– 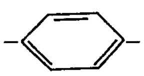 –$CH_3$), die in nahezu quantitativen Ausbeuten zu den gewünschten Keten-O,O-acetalen der Formel I führt, zumal von α-Halogenacetalen bekannt ist, dass eine Abspaltung von HCl nur schwer und unter drastischen Bedingungen möglich ist.

Die neuen Keten-O,O-acetale der Formel I eignen sich hervorragend zur Herstellung der bekannten Keten-O,N-acetale der Formel II.

Auf diese Weise ist es möglich, die weitgehend reinen E- oder Z-Isomere der Keten-O,N-acetale der Formel II zu erhalten, die ohne weitere Reinigung direkt als Fungizide verwendet werden können.

*Beispiele:*

Herstellung der Ausgangsverbindungen:

A1. Tosylierung von 1,1-Bis-(2,4-dichlorphenoxy)-3,3-dimethylbutan-2-ol.

42,4 g (0,1 mol) 1,1-Bis-(2,4-dichlorphenoxy)-3,3-dimethylbutan-2-ol werden in 200 ml absolutem Tetrahydrofuran (THF bei –10°C mit der equimolaren Menge einer Lösung von n-Butyllithium in n-Hexan umgesetzt. Anschliessend gibt man 19,1 g (0,1 mol) p-Toluolsulfonsäurechlorid zu, lässt auf Raumtemperatur (20°C) auftauen, rührt ca. 1 h nach, destilliert das Lösungsmittel im Vakuum ab, nimmt in Essigester auf und wäscht die organische Phase zweimal mit Wasser. Nach dem Trocknen und Einengen der organischen Phase wird das Rohprodukt aus Cyclohexan/Essigester umkristallisiert.

Ausbeute: 51,8 g (95% d. Th.)

Schmp.: 87-88°C

$^1$H-NMR: δ = 1,25 (s, 9H); 2,4 (s, 3H); 4,85 (d, 1H); 6,05 (d, 1H); 6,5-7,9 (m, 10H).

A2. Tosylierung von 1,1-Bis-(4-dichlorphenoxy)-3,3-dimethylbutan-2-ol.

35,6 g (0,1 mol) 1,1-Bis-(4-dichlorphenoxy)-3,3-dimethylbutan-2-ol werden in 200 ml absolutem Tetrahydrofuran mit 3 g (0,1 mol) Natriumhydrid (80%ig) versetzt und auf 40°C erhitzt. Sobald keine Gasentwicklung mehr stattfindet, gibt man 19,1 g (0,1 mol) p-Toluolsulfonsäurechlorid zu, rührt 1 h bei Raumtemperatur nach, hydrolysiert anschliessend mit wenig Wasser und engt in Vakuum ein. Der Rückstand wird in Essigester aufgenommen, zweimal mit Wasser gewaschen, getrocknet und erneut im Vakuum eingeengt. Das verbleibende Rohprodukt wird aus Essigester/Cyclohexan umkristallisiert.

Ausbeute: 41,7 g (82% d. Th.)

Schmp.: 97°C

$^1$H-NMR: δ = 1,25 (s, 9H); 2,3 (s, 3H); 4,8 (d, 1H); 5,8 (d, 1H); 6,5-7,9 (m 12H).

Nach dem gleichen Verfahren wurden folgende Verbindungen hergestellt:

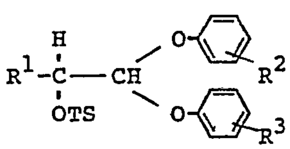

TS: 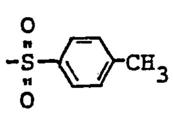

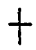 :tertiär-Butyl

| $R^1$ 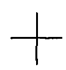 | $R^2$=$R^3$ | Schmp. (in °C) | $^1$H-NMR-Daten -Werte in $CDCl_3$ |
|---|---|---|---|
| 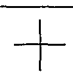 | 2-Cl | 79 | δ=1,2 (s, 9H); 2,3 (s, 3H); 4,9 (d, 1H); 6,1 (d, 1H); 6,5-7,9 (m, 12H) |
| 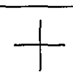 | 4-Br | 93- 95 | δ=1,2 (s, 9H); 2,3 (s, 3H); 4,8 (d, 1H); 5,8 (d, 1H); 6,5-7,9 (m, 12H) |
| 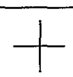 | 3,5 $Cl_2$ | 124-126 | δ=1,2 (s, 9H); 2,4 (s, 3H); 4,85 (d, 1H); 5,8 (d, 1H); 6,6-7,9 (m, 10H) |
| 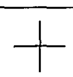 | 2,4,5 $Cl_3$ | 116-118 | δ=1,2 (s, 9H); 2,35 (s, 3H); 4,85 (s, 1H); 5,95 (s, 1H); 6,8-7,9 (m, 8H) |

| $R^1$ 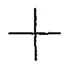 | $R^2=R^3$ | Schmp. (in °C) | $^1$H-NMR-Daten -Werte in $CDCl_3$ |
|---|---|---|---|
| 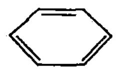 | $R^2$=4-Cl; $R^3$=2-Cl | 108-110 | δ=2,3 (s, 3H); 5,7-6 (m, 2H); 6,5-7,8 (m, 12H) |
| 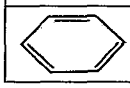 | $R^2$=2,4-$Cl_2$; $R^3$=4-Cl | | |

Herstellung der Keten-O,O-acetale.

1. 1,1-Bis-(2,4-dichlorphenoxy)-3,3-dimethylbuten-1.

54,6 g (0,1 mol) Tosylat des 1,1-Bis-(2,4-dichlorphenoxy)-3,3-dimethylbutan-2-ols werden in 200 ml trockenem Dimethylsulfoxid mit der equimolaren Menge Kalium-tert.-butanolat versetzt. Nach 30 min. Rühren bei Raumtemperatur hydrolysiert man mit Wasser, extrahiert zweimal mit dem gleichen Volumen Essigsäureethylester, trocknet und destilliert das Lösungsmittel im Vakuum ab.

Ausbeute: 38,57 g (95% d. Th.)

Sdp.: 175-176°C/0,5 mbar

$^1$H-NMR: δ = 1,2 (s, 9H); 4,85 (s, 1H); 7,73 (m, 6H).

2. 1,1-Bis-(4-chlorphenoxy)-3,3-dimethylbuten-1.

47,8 g (0,1 mol) Tosylat des 1,1-Bis-(4-chlorphenoxy)-3,3-dimethylbutan-2-ols werden in 200 ml trockenem Dimethylsulfoxid mit 39 g (0,5 mol) Natriumsulfid versetzt und so lange bei 120°C gerührt, bis keine Ausgangsverbindung mittels Hochdruckflüssigkeitschromatographie (HPLC) mehr nachweisbar ist. Anschliessend kühlt man ab, versetzt mit Wasser und extrahiert zweimal mit dem gleichen Volumen Essigester, trocknet und destilliert das Lösungsmittel im Vakuum ab.

Ausbeute: 25,3 g (75% d. Th.)

$^1$H-NMR: δ = 1,2 (s, 9H); 4,8 (s, 1H); 6,8-7,4 (m, 8H).

Nach dem gleichen Verfahren wurden folgende Verbindungen hergestellt:

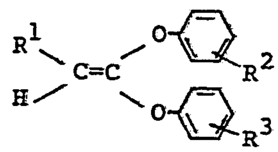

| $R^1$ | $R^2=R^3$ | $^1$H-NMR-Daten -Werte in $CDCl_3$ |
|---|---|---|
| 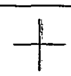 | 2-Cl | δ=1,2 (s, 9H); 4,8 (s, 1H); 6,8-7,4 (m, 8H) |
| 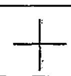 | 4-Br | δ=1,15 (s, 9H); 4,8 (s, 1H); 6,7-7,5 (m, 8H) |
| 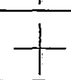 | 3,5 $Cl_2$ | δ=1,15 (s, 9H); 4,95 (s, 1H); 6,8-7,1 (m, 6H) |
| 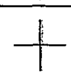 | 2,4,5-$Cl_3$ | δ=1,2 (s, 9H); 4,95 (s, 1H); 7,25-7,5 (m, 4H) |
| 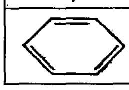 | $R^2$=2,4-$Cl_2$; $R^3$=4-Cl | δ=5,8 (s, 1H); 7,1-7,8 (m, 12H); 5,9 (s, 1H); 7,1-7,8 (m, 12H); E/Z-Isomerengemisch |

Verwendung der Keten-O,O-acetale der Formel I zur Herstellung der aus EP Nr. 56125 bekannten Keten-O,N-acetale der Formel II.

*Vorschrift:*

Herstellung von Z-1-(1,2,4-Triazol-1-yl)-2-(2,4-dichlorphenoxy)-3,3-dimethylbuten-1.

30,4 g (0,075 mol) 1,1-Bis-(2,4-dichlorphenoxy)-3,3-dimethylbuten-1 und 5,2 g (0,075 mol) Triazol werden zusammen auf 180-185°C erhitzt. Sobald die Reaktionsmischung homogen erscheint und sich rötlich-braun verfärbt hat, wird mittels Hochdruckflüssigkeitschromatographie (HPLC) der Gehalt an Ausgangsverbindung bestimmt. Hat die Reaktion den gewünschten Umsetzungsgrad erreicht, kühlt man ab, nimmt in 200 ml Hexan/Essigester (1:1) auf, wäscht 2-3mal mit dem gleichen Volumen verdünnter Natronlauge (5%), trocknet mit $Na_2SO_4$ und destilliert das Lösungsmittel im Vakuum ab. Man erhält 22,2 g Keten-O,N-acetal. Isomerenreinheit: Z-Isomeres 85%, bestimmt mittels $^1$H-NMR und HPLC.

In entsprechender Weise können die folgenden Verbindungen unter Verwendung der entsprechenden Keten-O,O-acetale hergestellt werden.

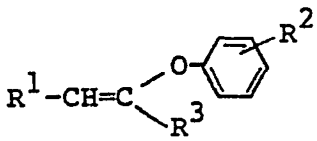

| Nr. | $R^1$ | $R^2$ | $R^3$ | Phys. Daten $^1$H-NMR<br>Reinheit des Isomeren -Werte in $CDCl_3$ |
|---|---|---|---|---|
| 1 | $(CH_3)_3C-$ | 2,4-$Cl_2$ | 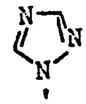 | 85% Z-Isomeres<br>δ=1,25 (s, 9H); 5,85 (s, 1H); 6,7-7 (m, 3H); 7,85 (s, 1H); 8,1 (s, 1H) |
| 2 | $(CH_3)_3C-$ | 4-Cl | 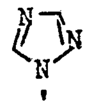 | 90% Z-Isomeres<br>δ=1,25 (s, 9H); 5,9 (s, 1H); 6,8-7,4 (m, 4H); 7,95 (s, 1H); 8,2 (s, 1H) |
| 3 | $(CH_3)_3C-$ | 2-Cl | 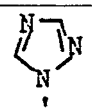 | 90% Z-Isomeres<br>δ=1,15 (s, 9H); 5,9 (s, 1H); 6,75-7,5 (m, 4H); 7,9 (s, 1H); 8,15 (s, 1H) |
| 4 | $(CH_3)_3C-$ | 4-Cl | 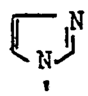 | 80% Z-Isomeres<br>δ=1,25 (s, 9H); 5,25 (s, 1H); 6,7-7,7 (m, 7H) |
| 5 | $(CH_3)_3C-$ | 2,4-$Cl_2$ | 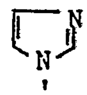 | 75% Z-Isomeres<br>δ=1,3 (s, 9H); 5,4 (s, 1H); 6,8-7,8 (m, 6H) |
| 6 | $(CH_3)_3C-$ | 2-Cl | 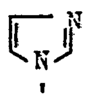 | 80% Z-Isomeres<br>δ=1,25 (s, 9H); 5,35 (s, 1H); 6,7-7,8 (m, 7H) |
| 7 | $(CH_3)_3C-$ | 4-Cl | 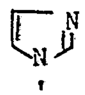 | 80% Z-Isomeres<br>δ=1,35 (s, 9H); 5,4 (s, 1H); 6,8-8,1 (m, 9H) |

**Patentansprüche** für die Vertragsstaaten BE, CH, DE, FR, GB, IT, LI, NL, SE

1. Keten-O,O-acetale der Formel I:

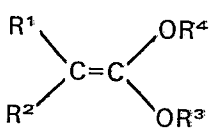

(I)

in der:

$R^1$ einen tertiären Alkylrest mit 4 bis 6 C-Atomen oder einen durch Halogen substituierten Phenylrest;

$R^2$ Wasserstoff;

$R^3$ und $R^4$ unabhängig voneinander einen gegebenenfalls durch Halogen, Alkoxy, Alkyl, Phenyl, Phenoxy, Cyano, Nitro, Trifluormethyl substituierten Phenylrest bedeuten.

2. Verfahren zur Herstellung eines Keten-O,O-acetals der Formel I gemäss Anspruch 1, dadurch gekennzeichnet, dass man:

a) ein α-Hydroxyacetal der Formel II:

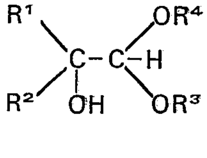

(II)

in der:

$R^1$ bis $R^4$ die im Anspruch 1 genannten Bedeutungen haben mit der Verbindung:

Cl–Z

zu der Verbindung der Formel III:

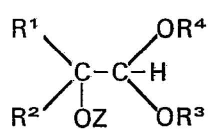

(III)

umsetzt, in der Z den Rest $-SO_2-R$ bedeutet, wobei R Alkyl, Haloalkyl oder gegebenenfalls mehrfach substituiertes Phenyl bedeutet, und

b) die Verbindung der Formel III mit einer basischen Verbindung zu dem Keten-O,O-acetal der Formel I gemäss Anspruch 1 umsetzt.

**Patentanspruch** für Vertragsstaat: AT

1. Verfahren zur Herstellung eines Keten-O,O-acetals der Formel I:

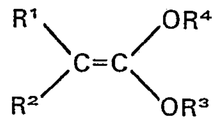

(I)

in der:

$R^1$ einen tertiären Alkylrest mit 4 bis 6 C-Atomen oder einen durch Halogen substituierten Phenylrest;

$R^2$ Wasserstoff;

$R^3$ und $R^4$ unabhängig voneinander einen gegebenenfalls durch Halogen, Alkoxy, Alkyl, Phenyl, Phenoxy, Cyano, Nitro, Trifluormethyl substituierten Phenylrest bedeuten, dadurch gekennzeichnet, dass man:

a) ein α-Hydroxyacetal der Formel II:

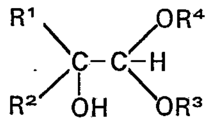

(II)

in der:

$R^1$ bis $R^4$ die im Anspruch 1 genannten Bedeutungen haben mit der Verbindung:

Cl−Z

zu der Verbindung der Formel III:

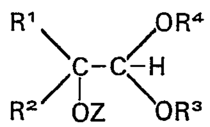

(III)

umsetzt, in der Z den Rest $-SO_2-R$ bedeutet, wobei R Alkyl, Haloalkyl oder gegebenenfalls mehrfach substituiertes Phenyl bedeutet, und

b) die Verbindung der Formel III mit einer basischen Verbindung zu dem Keten-O,O-acetal der Formel I umsetzt.

**Claims** for the Contracting States BE, CH, DE, FR, GB, IT, LI, NL, SE

1. A ketene O,O-acetal of the formula I:

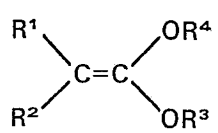

(I)

where:

$R^1$ is a tertiary alkyl radical of 4 to 6 carbon atoms or halogen-substituted phenyl;

$R^2$ is hydrogen, and

$R^3$ and $R^4$ independently of one another are phenyl which is unsubstituted or substituted by halogen, alkoxy, alkyl, phenyl, phenoxy, cyano, nitro or trifluoromethyl.

2. A process for the preparation of a ketene O,O-acetal of the formula I as claimed in Claim 1, wherein:

a) an α-hydroxyacetal of the formula II:

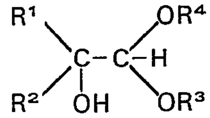

(II)

where:

$R^1$, $R^2$, $R^3$ and $R^4$ have the meanings given in Claim 1, is reacted with a compound:

Cl−Z

to give a compound of the formula III:

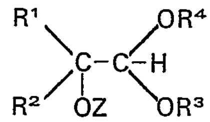

(III)

where Z is a radical $-SO_2-R$, where R is alkyl, haloalkyl or optionally polysubstituted phenyl, and

b) the compound of the formula III is reacted with a basic compound to give the ketene O,O-acetal of the formula I as claimed in Claim 1.

**Claim** for the Contracting State: AT

A process for the preparation of a ketene O,O-acetal of the formula I:

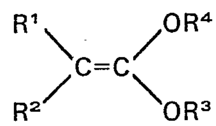

(I)

where:

$R^1$ is a tertiary alkyl radical of 4 to 6 carbon atoms or halogen-substituted phenyl;

$R^2$ is hydrogen, and

$R^3$ and $R^4$ independently of one another are phenyl which is unsubstituted or substituted by halogen, alkoxy, alkyl, phenyl, phenoxy, cyano, nitro or trifluoromethyl, wherein:

a) an α-hydroxyacetal of the formula II:

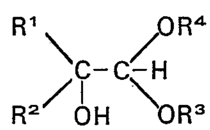

(II)

where $R^1$, $R^2$, $R^3$ and $R^4$ have the meanings given in Claim 1, is reacted with a compound:

Cl−Z

to give a compound of the formula III:

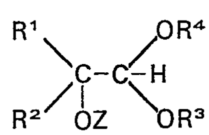

(III)

where Z is the radical $-SO_2-R$, where R is alkyl, haloalkyl or optionally polysubstituted phenyl, and

b) the compound of the formula III is reacted with a basic compound to give the ketene O,O-acetal of the formula I.

**Revendications** pour les Etats contractants BE, CH, DE, FR, GB, IT, LI, NL, SE

1. O,O-Acétale de cétènes de formule I:

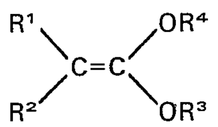

(I)

dans laquelle:

$R^1$ représente un groupe tert.-alkyle en C4-C6 ou un groupe phényle substitué par des halogènes,

$R^2$ représente l'hydrogène;

$R^3$ et $R^4$ représentent, chacun, indépendamment l'un de l'autre, un groupe phényle éventuellement substitué par des halogènes, des groupes alcoxy, alkyle, phényle, phénoxy, cyano, nitro, trifluorométhyle.

2. Procédé de préparation d'un O,O-acétale de cétènes de formule I selon la revendication 1, caractérisé par le fait que:

a) on fait réagir un α-hydroxyacétal de formule II:

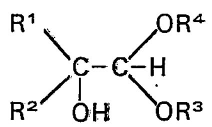

(II)

dans laquelle les symboles $R^1$ à $R^4$ ont les significations indiquées dans la revendication 1, avec le composé:

Cl–Z

ce qui donne le composé de formule III:

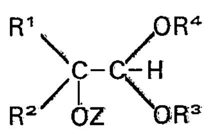

(III)

dans laquelle Z représente le groupe $-SO_2-R$ dans lequel R représente un groupe alkyle, halogénoalkyle ou phényle éventuellement polysubstitué et

b) on convertit le composé de formule III, à l'aide d'un composé basique, en O,O-acétate de cétènes de formule selon la revendication 1.

**Revendication** pour l'Etat contractant: AT

Procédé de préparation d'un O,O-acétale de cétènes de formule I:

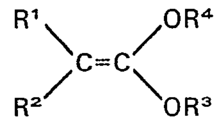

(I)

dans laquelle:

$R^1$ représente un groupe tert.-alkyle en C4-C6 ou un groupe phényle substitué par des halogènes,

$R^2$ représente l'hydrogène,

$R^3$ et $R^4$ représentent, chacun, indépendamment l'un de l'autre, un groupe phényle éventuellement substitué par des halogènes, des groupes alcoxy, alkyle, phényle, phénoxy, cyano, nitro, trifluorométhyle, caractérisé par le fait que:

a) on fait réagir un α-hydroxyacétal de formule II:

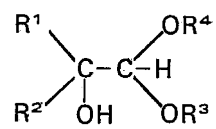

(II)

dans laquelle les symboles $R^1$ à $R^4$ ont les significations indiquées ci-dessus, avec le composé:

Cl–Z

ce qui donne le composé de formule III:

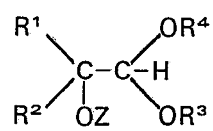

(III)

dans laquelle Z représente le groupe $-SO_2-R$ dans lequel R représente un groupe alkyle, halogénoalkyle ou phényle éventuellement polysubstitué et

b) on convertit le composé de formule III, à l'aide d'un composé basique, en O,O-acétale de cétènes de formule I.